# FASCICULE DE BREVET EUROPEEN

(11) **EP 1 131 039 B1**
(45) Date de publication et mention de la délivrance du brevet: **22.06.2005**
(21) Numéro de dépôt: 99972094.9
(22) Date de dépôt: 10.11.1999
(51) Int. Cl.: A61K 7/00

(54) **UTILISATION D'UNE SUBSTANCE SE LIANT AU RECEPTEUR PERIPHERIQUE DES BENZODIAZEPINES DANS LE TRAITEMENT DES STRESS CUTANES**
VERWENDUNG EINER SICH AN PERIPHERE BENZODIAZEPINREZEPTOREN BINDENDEN SUBSTANZ ZUR BEHANDLUNG VON HAUTSTRESS
USE OF A SUBSTANCE BINDING WITH THE PERIPHERAL BENZODIAZEPIN RECEPTOR FOR TREATING SKIN STRESS

(30) Priorité: 17.11.1998 FR 9814387
(43) Date de publication de la demande: 12.09.2001
(73) Titulaire: Sanofi-Aventis, 75013 Paris (FR)
(72) Inventeur: Casellas, Pierre, 34090 Montpellier (FR); Derocq, Jean-Marie, 34570 Murviel les Montpellier (FR)
(74) Mandataire: Monain, Patrice
(86) Numéro de dépôt international: PCT/FR1999/002761
(87) Numéro de publication internationale: WO 2000/028947

(56) Documents cités:
- LEVY: "A psychosomatic approach to the management of recalcitrant dermatoses" PSYCHOSOMATICS, vol. 4, no. 6, novembre 1963 (1963-11), pages 334-337, XP000878884
- ORMFA ET AL.: "Ricerche preliminari clinico psicologiche su di un nuovo derivato benzodiazepinico (Nobrium) in dermatologia" GIORNALE ITALIANO DI DERMATOLOGIA. MINERVA DERMATOLOGICA, vol. 45, no. 5, mai 1970 (1970-05), pages 325-339, XP000878885
- CARAYON ET AL.: "Involvement of peripheral benzodiazepine receptors in the protection..." BLOOD, vol. 87, no. 8, avril 1996 (1996-04), pages 3170-3178, XP002091195 cité dans la demande
- BOH: "Role of reactive oxygen species in dermatologic diseases" CLINICS IN DERMATOLOGY, vol. 14, no. 4, juillet 1996 (1996-07), pages 343-352, XP000878889

## Description

La présente invention concerne une composition contenant un ligand des récepteurs périphériques des benzodiazépines pour une utilisation par voie topique.

L'invention concerne l'utilisation d'une substance se liant spécifiquement au récepteur périphérique des benzodiazépines (PBR) pour la fabrication d'une composition pour la prophylaxie ou le traitement des stress cutanés.

L'invention concerne également les compositions contenant ces substances. Ces compositions peuvent être cosmétiques ou pharmaceutiques, en particulier dermatologiques topiques.

Par stress cutané, on entend les différentes situations qui pourraient provoquer des dommages en particulier au niveau de l'épiderme quelque soit l'agent le provoquant. Cet agent peut être interne et/ou externe à l'organisme comme un agent chimique ou radicalaire ou bien externe comme un rayonnement ultraviolet.

La composition selon l'invention est donc destinée à prévenir et à lutter contre les irritations cutanées, les dartres, les érythèmes, les sensations dysesthésiques, les sensations d'échauffement, les prurits de la peau et/ou des muqueuses, le vieillissement et peut aussi être utilisée dans les désordres cutanés tels que, par exemple, le psoriasis, les maladies prurigineuses, l'herpès, les photodermatoses, les dermatites atopiques, les dermatites de contact, les lichens, les prurigos, les prurits, les piqures d'insectes, dans les fibroses et autres troubles de la maturation des collagènes, dans les désordres immunologiques ou encore dans des affections dermatologiques comme l'eczéma.

Le ligand PBR, également appelé "substance", contenu dans la composition peut-être un composé non peptidique, un peptide, un extrait de cellules ou de tissus d'origine animale ou végétale ou un produit obtenu par fermentation d'un micro-organisme, par exemple d'une bactérie ou d'un champignon.

De nombreux ligands PBR sont décrits dans la littérature. On peut citer, à titre d'exemple, le Ro 5-4864 ou chlorodiazépam, le Ro 5-2807 ou diazépam, le PK 11195 ou on pourra se référer à l'article Peripheral Benzodiazepine Receptors, Ch. III, J. J. Bourguignon, Ed. E. Giesen - Crouse, Academic Press.

Le PBR est une protéine de 18 KD située sur la membrane externe des mitochondries des tissus périphériques. Il est constitué de cinq domaines transmembranaires et d'une partie carboxyterminale dirigée vers le cytosol. Plusieurs fonctions sont attribuées au PBR selon la nature du tissus considéré : régulation de la stéroïdogénèse, biosynthèse de l'hème, différenciation et croissance cellulaire, contrôle de la respiration mitochondriale (Krueger KE, Biochimica and Biophysica Acta 1995, *1241*, 453-470). Bien que sa fonction précise n'ait pas encore été complètement élucidée, plusieurs données expérimentales récentes suggèrent que PBR pourrait jouer un rôle fondamental dans la régulation des processus de mort cellulaire programmée et dans la protection anti-radicalaire.

Il a été montré que PBR est en effet étroitement associé au niveau de la mitochondrie avec des protéines régulatrices de l'apoptose telles que Bcl2 qui prévient la rupture du potentiel de membrane mitochondriale empêchant ainsi l'apoptose induite notamment par la production de radicaux oxygénés réactifs (Marchetti P. *et al*, J. Exp. Med. 1996, *184*, 1155-1160); (Marchetti P. *et al*, J. Immunol. 1996, *157*, 4830-4836).

Dans le cadre de la présente invention, le rôle protecteur anti-radicalaire de PBR a été directement observé sur des cellules d'origine hématopoïétique pour lesquelles une corrélation étroite entre la densité de PBR et la protection anti-radicalaire a été mise en évidence. De plus, dans cette même étude, il a été démontré que la transfection de PBR sur des cellules dépourvues de ce récepteur confère une protection contre les dommages causés par des espèces oxygénées (Carayon P. *et al*, Blood 1996, *87*, 3170-3178).

Plusieurs données de la littérature indiquent que PBR jouerait un rôle important dans la régulation des processus apoptotiques et la protection des cellules *vis-à-vis* des dommages causés par les radicaux libres.

Des études phylogéniques récentes renforcent cette nouvelle notion que PBR agit en tant que modulateur d'apoptose impliqué dans des fonctions antioxydantes. Des similarités significatives existent en effet entre PBR et la protéine CrtK de *Rhodobacter sphaeroides,* une bactérie photosynthétique. Cette protéine bactérienne qui fonctionne comme un détecteur d'oxygène photosensible, régule l'expression des gènes impliqués dans la photosynthèse en réponse à des modifications environnementales de la tension en oxygène et de l'intensité lumineuse. La comparaison entre PBR et

CrtK révèle une identité de 35 % et une conservation de séquence entre ces deux protéines qui ont divergé dans la phylogénie il y a deux milliards d'années. Cette homologie suggère une fonction hautement spécialisée et conservée de PBR qui semble similaire à celle de CrtK dans la bactérie. En effet, il a été démontré récemment que le PBR de mammifère transfecté chez des mutants *Rhodobacter* CrtK complémente la fonction détecteur d'oxygène de CrTK. Ainsi, cette étude suggère un rôle clé de PBR dans la transduction des signaux dépendant de l'oxygène (Yeliseev AA., *et al,* Proc. Natl. Acad. Sci. 1997, *94,* 5101-5106).

Cependant, à ce jour, aucune substance n'a jamais été précisément indiquée comme ligand spécifique des récepteurs PBR cutanés.

A plus forte raison, aucune substance active par voie topique, et se liant spécifiquement aux récepteurs PBR n'a jamais été décrite dans la littérature.

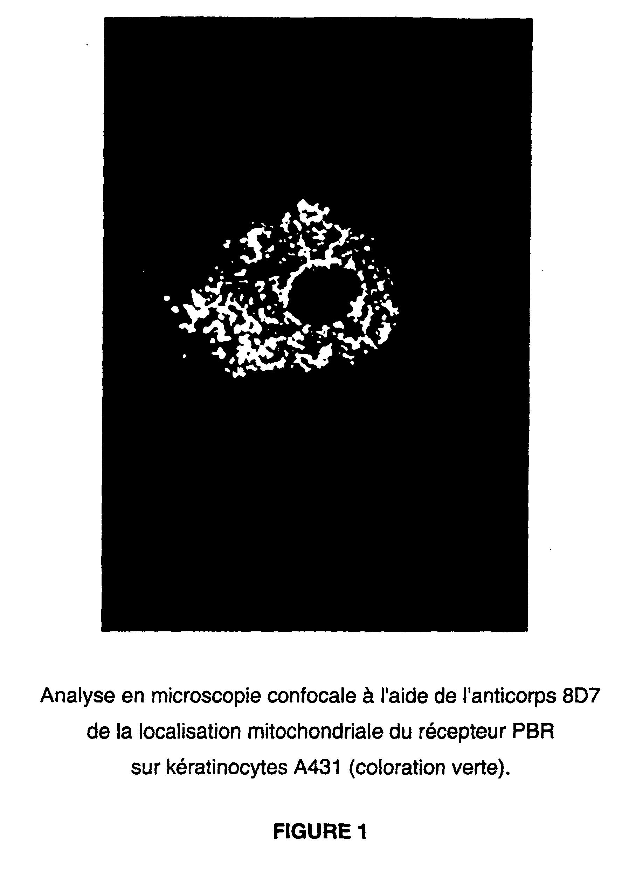

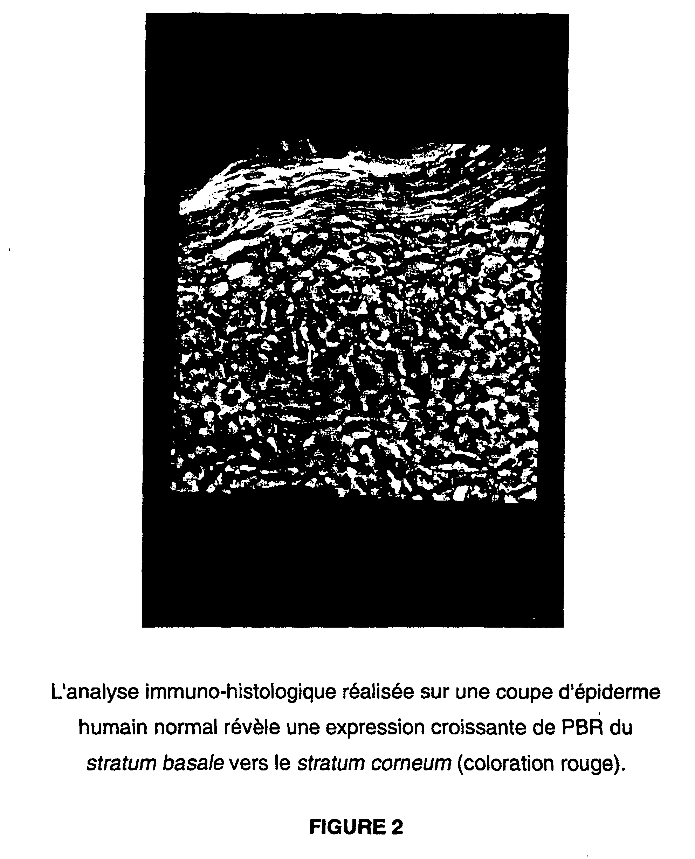

Il a été maintenant montré, dans le cadre de la présente invention, que PBR est abondamment exprimé au niveau de la peau sur les différents compartiments cellulaires qui la composent : kératinocytes, cellules de Langerhans, follicules pileux et cellules endothéliales de la vasculature dermique. Au niveau de la peau, l'expression de PBR suit un gradient croissant de la couche basale vers la couche cornée. Cette organisation remarquable qui privilégie les cellules différenciées de l'épiderme les plus exposées aux stress externes revêt sans doute une signification physiologique de première importance pour la protection des zones de l'épiderme les plus vulnérables. Les études subcellulaires réalisées en microscopie confocale indiquent comme attendu une colocalisation de PBR avec Bcl2 au niveau de la mitochondrie. Les études histologiques sur coupe de peau ont permis de mettre en évidence une répartition surprenante de PBR (Figures 1 et 2).

En effet, l'expression de ce récepteur au niveau de l'épiderme suit un gradient de densité croissant des couches basales vers les couches les plus différenciées de kératinocytes. Cette distribution spatiale très organisée qui privilégie en terme de densité les cellules de l'épiderme les plus externes et donc les plus exposées, laisse supposer que PBR au niveau de la peau pourrait représenter un système de protection naturelle contre les radicaux libres générés par l'exposition aux rayonnements ultraviolets. L'observation concommitante que la distribution de la protéine antiapoptotique Bcl2 obéit à un gradient strictement inverse suggère un rôle compensatoire de PBR pour la préservation des cellules les plus différenciées.

L'ensemble de ces données qui suggèrent une fonction protectrice du PBR, plus particulièrement au niveau de l'épiderme, a conduit à la mise en évidence de ligands naturels ou de synthèse montrant que leur interaction avec PBR pouvait être bénéfique dans différentes situations de stress cutané provoqué par des agents chimiques ou radicalaires ou encore consécutifs à une exposition UV.

Ainsi, selon l'un de ses aspects, la présente invention concerne l'utilisation d'un ligand spécifique du PBR, le Ro 5-4864 dans les stress cutanés. Ce ligand est un agoniste du PBR.

Selon un autre aspect de l'invention et sur la base de ces observations, un screening visant à rechercher des ligands naturels de PBR a été entrepris et a permis d'isoler plusieurs fractions capables d'interagir avec ce récepteur. L'effet potentiellement protecteur de ces ligands naturels a ensuite été évalué dans différents tests induisants un stress cutané et notamment des tests d'érythèmes cutanés induits par irradiations

UV. Les propriétés anti-radicalaires ainsi que les capacités de réparation cutanée ont également été recherchées.

Des essais biochimiques et pharmacologiques ont été utilisés pour mettre en évidence l'acitivité et l'intérêt des substances dans divers stress cutanés.

Les essais réalisés avec PBR ont pour but de montrer son implication potentielle dans la régulation des processus apoptotiques et la préservation des cellules cutanées vis-à-vis de différents stress délétères.

### EXEMPLE 1

### Eudes immuno-histologiques de localisation de PBR au niveau cutané

A l'aide de l'anticorps spécifique anti-PBR, Ac 8D7 (mAb de souris anti-PBR, isotype IgG1, Dussossoy *et al*., Cytometry, 1996, 24:39-48), une analyse par Western Blot a permis de mettre en évidence la présence abondante de PBR sur six lignées différentes de kératinocytes humains ainsi que sur la peau humaine normale (Figure 1). Au niveau subcellulaire, les analyses réalisées en microscopie confocale confirment sur les kératinocytes une colocalisation de PBR au niveau mitochondrial (Figure 2).

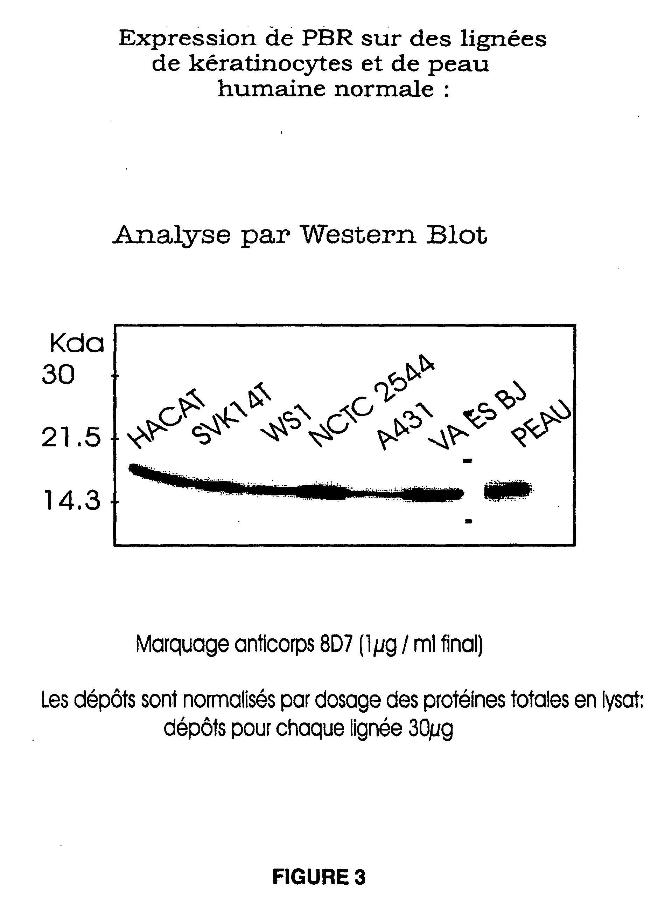

Une étude immuno-histologique réalisée sur une coupe d'épiderme humain normal à l'aide du même anticorps révèle un ordonnancement très particulier puisque l'expression de PBR augmente du *stratum basale* vers le *stratum corneum.* Ce récepteur est donc abondament présent sur les kératinocytes les plus différenciés situés directement sous le *stratum corneum* (Figure 3).

### EXEMPLE 2

### Etudes de liaison et de spécificité

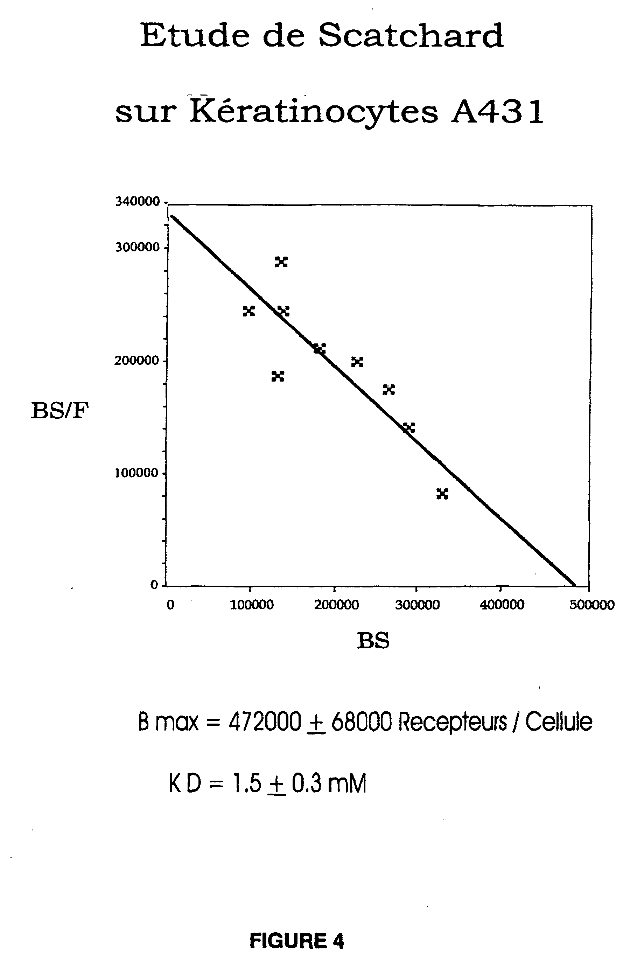

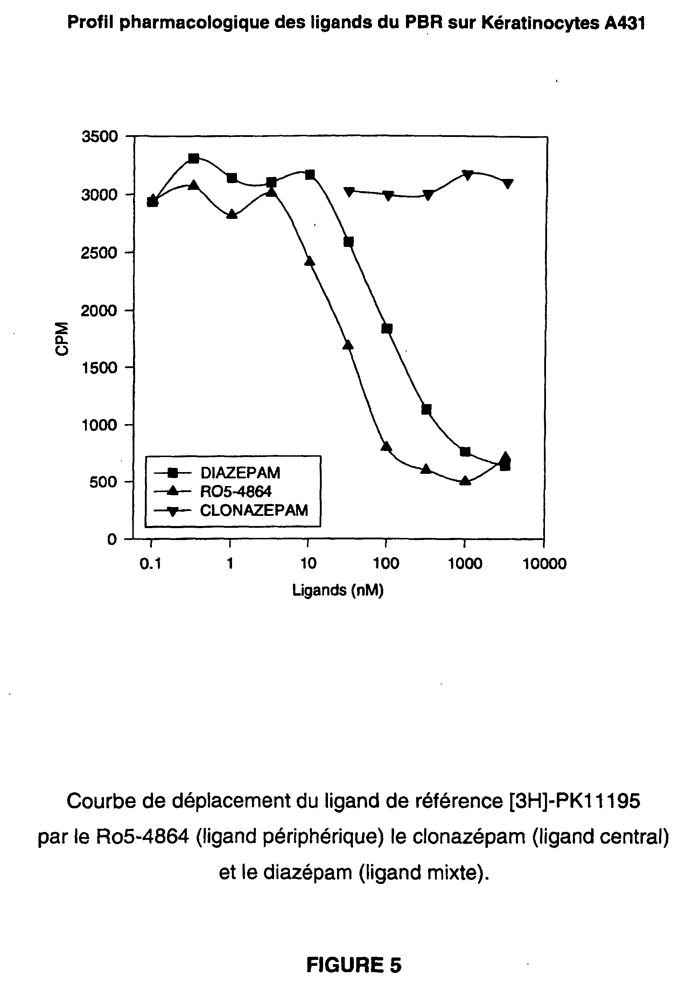

Les études de liaison ou binding ont été réalisées sur la lignée de kératinocytes A-431 (carcinome épidermoïde humain (ATCC, CRL-1555)) par déplacement du ligand de référence [³H]-PK11195. L'analyse de Scatchard indique un seul site de fixation, une densité d'environ 470.000 récepteurs par cellule et une forte affinité du ligand (KD = 1,5 nM) (Figure 4). La spécificité du binding sur le récepteur périphérique porté par les kératinocytes est attestée par les études pharmacologiques qui montrent une efficacité décroissante du déplacement du ligand périphérique de référence (PK 11195) par les ligands suivants :
Ro 5-4864 = (CI₅₀ ≈ 25 nM) > diazepam (CI₅₀ ≈ 100 nM) >>> clonazepam (inactif à 3200 nM). Il est rappelé que ce dernier est un ligand du récepteur central des benzodiazépines, le diazepam est mixte, le Ro 5-4864 est spécifique du PBR (Figure 5).

### EXEMPLE 3

### Implication du PBR dans la protection contre les radicaux oxygénés

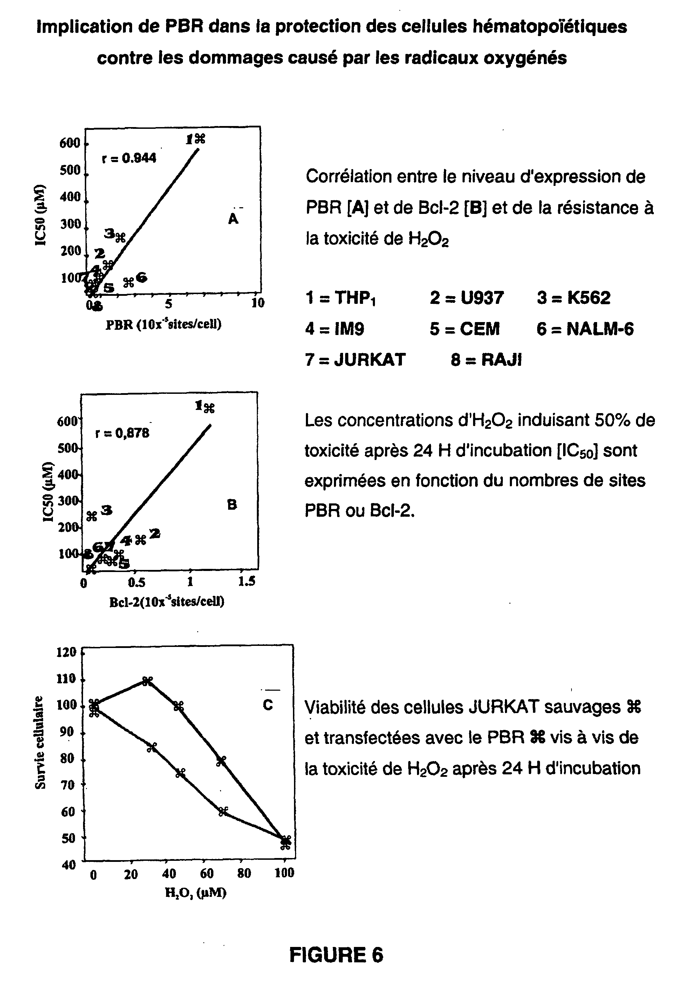

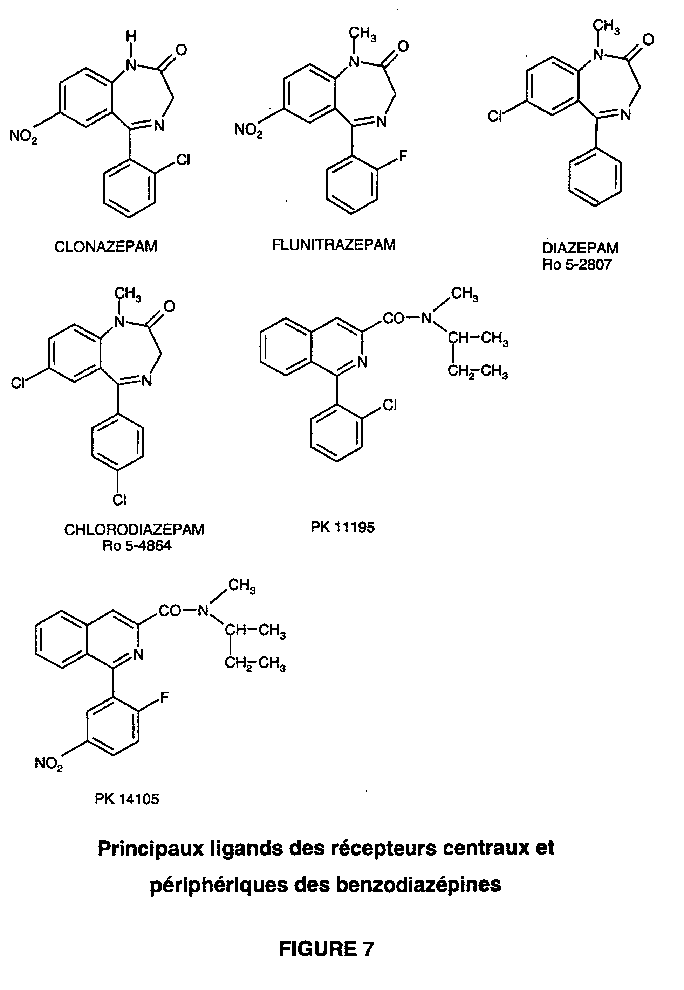

Deux types d'expériences sont décrites à la Figure 6. La première a consisté à comparer différentes lignées d'origine lymphoïde ou myéloïde pour leur capacité à résister à la toxicité des radicaux oxygénés en relation avec leur niveau d'expression de PBR. Les résultats indiquent une très forte corrélation entre le nombre de sites PBR par cellule et la résistance à la toxicité induite par l'H₂O₂. Il existe également une corrélation similaire lorsque l'on considère cette fois le niveau d'expression de Bcl2, protéine connue pour protéger les cellules des dommages oxydatifs. Cette donnée, jointe au fait que Bcl2 et PBR sont deux protéines localisées sur la membrane externe des mitochondries, suggère qu'elles pourraient présenter des fonctions communes dans la protection cellulaire. De façon intéressante, alors que l'expression de PBR suit un gradient de densité croissant de la couche basale vers la limite de la couche cornée, les données de la littérature indiquent un phénomène inverse pour l'expression de Bcl2, suggérant qu'au cours de la différenciation des kératinocytes, PBR pourrait prendre le "relais" de Bcl2 en ce qui concerne les fonctions de protection anti-radicalaire.

Dans la deuxième expérience, le rôle possible de PBR dans la protection *vis-à-vis* de la toxicité des radicaux libres est renforcé par la démonstration de la meilleure viabilité, en présence d'H₂O₂, de cellules Jurkat transfectées PBR+ en comparaison avec des cellules homologues PBR-.

### EXEMPLE 4

L'activité anti-apoptotique des actifs a été mesurée sur des kératinocytes humains et des fibroblastes (ATCC) qui ont été ensemencés dans des boites de Pétri de 35 mm (5 X 10⁵ cellules/puits) dans le DMEM (Dubelco's Mode Eagle Medium) additionné de 10 % de sérum de veau foetal et laisser proliférer jusqu'à confluence. Ce milieu de culture est ensuite aspiré, les cellules sont rincées et 0,1 % de sérum de veau foetal est ajouté en présence d'une solution saline. Des concentrations croissantes de la substance à étudier sont additionnées. Vingt quatre heures plus tard, l'apoptose est mesurée avec une trousse de dosage ELISA (de l'anglais "enzyme-linked immunosorbent assay").

Des kératinocytes ont été soumis à des rayonnements ultraviolets de type B (UVB) à la dose de 250J/m² pendant 16 heures (J. Invest. Dermatol. 1995, *104* : 922-927). En présence du ligand PBR Ro 5-4864, il a été montré que l'on prévient les processus d'altérations cellulaires induits par l'irradiation dans une gamme de concentrations de ligand comprise entre 10 nM et 10 µM.

### EXEMPLE 5

L'effet photoprotecteur du ligand a été évalué par application par voie cutanée sur le cobaye albinos.

La voie topique cutanée est utilisée afin de reproduire les conditions d'utilisation chez l'homme.

Des cobayes Hartley, Charles River France, Saint Aubin lès Elbeuf, 76410 Cléon, France, sont utilisés.

Les animaux ont été rasés puis épilés sur les flancs antérieurs droit et gauche, 24 heures avant le début du traitement.

Les animaux ont été irradiés juste avant le premier traitement. L'énergie est vérifiée avant chaque irradiation réalisée sur les flancs droit et gauche dans le spectre UVB à une dose de 4000 mJ/cm².

Le flanc droit des animaux a été traité avec 0,2 ml de solution du ligand immédiatement après irradiation puis 2 et 5 heures après irradiation. Le flanc gauche ne sera pas traité.

Une lampe à vapeur de Xéron Haute pression (IDEM 300) produira l'irradiation.

Les lectures des réactions locales sont faites avant traitement, puis 5 et 24 heures après irradiation.

Erythème et oedème ont été évalués comme suit :
Erythème
   0 pas d'érythème ; 1 érythème très faible, à peine perceptible ; 2 érythème net, rose faible ; 3 érythème net, rouge franc ; 4 érythème particulièrement intense
Oedème
   0 pas d'oedème ; 1 très léger oedème (à peine visible) ; 2 léger oedème (contours bien définis et gonflement apparent) ; 3 oedème moyen (épaisseur d'environ 1 mm) ; 4 oedème grave (épaisseur supérieure à 1 mm et surface supérieure à la zone d'application)

Des exemples de ligands naturels du récepteur PBR produits par fermentation sont décrits ci-après avec leur activité.

Un criblage ou screening effectué sur des extraits de microorganismes conduit sur milieu solide ou liquide a permis de sélectionner trois souches de microorganismes (bactéries et champignons microscopiques).

Les deux souches sélectionnées après différentes études menées pour optimiser les conditions de production de quantités significatives d'extraits de cultures possédant de bonnes activités sur te test de mesure d'interaction avec le récepteur PBR ont pour références SRL 4988 et SRL 5186.

Les trois souches ci-dessus ont été déposées à la CNCM de l'Institut Pasteur : date de 27 août 1999 avec respectivement les numéros d'ordre I-2305, I-2306.

La souche SRL 4988 classée comme *Nocardia species,* isolée d'un échantillon de sol a pour caractères écologico-physiologiques, déterminés après culture de deux semaines à 28° C sur milieu ISP2 : phototrophe négatif, chimio-organotrophe, mésophile et halophile négatif. Elle est non mobile et présente des spires ouvertes, non verticillées.

La souche SRL 5186 classée comme *Streptomyces species,* isolée d'un échantillon de sol a pour caractères écologico-physiologiques, déterminés après culture de deux semaines à 28° C sur milieu ISP2 : phototrophe négatif, chimio-organotrophe, mésophile et halophile négatif. Elle est non mobile et présente des hyphes flexibles et biverticillées.

Après cultures sur milieu nutritif gélosé et plusieurs repiquages successifs qui permettent d'obtenir une culture abondante et pure, on fabrique un lot 0 de conservation de la souche mère puis des lots de semence primaire et secondaire.

Pour cela, on prépare une suspension de spores à partir d'une culture sur milieu nutritif gélosé en boîte de Pétri et d'un milieu de reprise ; ce milieu contient un cryoprotecteur permettant d'assurer une bonne viabilité des spores lors de la conservation par congélation.

La suspension de spores obtenue est répartie dans des cryotubes qui seront conservés à - 80° C : ces tubes constituent le lot 0.

En suivant le même protocole, mais à partir d'un tube du lot 0, on prépare un lot de semence primaire. Puis toujours suivant le même protocole, on prépare un lot de semence secondaire à partir d'un cryotube de semence primaire. La fabrication des lots de semence 0, 1 et 2 assure une accessibilité durable de la souche et donc de l'activité recherchée. Les cultures de ces trois souches permettant d'obtenir des ligands naturels du récepteur PBR peuvent être menées de manière proche avec les moyens de cultures aérobies habituels, c'est-à-dire milieux liquides en fermenteurs de toute capacité avec contrôle en ligne du pH et de l'aération.

### EXEMPLE A SRL 4988

A titre d'exemple de culture en fioles d'Erlenmeyer pour la souche SRL 4988 : un tube de semence secondaire est utilisé pour ensemencer des boîtes de Pétri préparées avec un milieu favorisant la sporulation des actinomycètes selon la composition :

| | |
|---|---|
| Glucose | 20 g |
| Soyoptim (SIO) | 10 g |
| CaCO₃ (OMYA) | 3g |
| Agar type E | 20 g |
| Eau distillée QSP | 1 l |

On fait incuber les cultures sur boites pendant 5 jours à 28° C. On obtient alors une suspension de spores en ajoutant dans chaque boite de Pétri, 10 ml d'un milieu liquide de composition :

| | |
|---|---|
| Glucose | 30 g |
| Soyoptim (SIO) | 10 g |
| Tryptone U.S.P. (BIOKAR) | 4 g |
| Extrait de levures (DIFCO) | 8 g |
| NaCl | 2,5 g |
| CaCO₃ | 5 g |
| Hydrolysat de caséine | 5 g |
| Peptone papaïnique de soja | 5 g |

dont le pH est ajusté à 7,0 avant stérilisation.

On utilise 5 ml des suspensions de spores pour inoculer des fioles stériles de 250 ml, garnies avec 50 ml du même milieu, qui constituent les précultures, mises à incuber en chambre chaude à 28° C sur un agitateur à étagères, ou dans un incubateur autonome, les vitesses de rotation sur l'un ou l'autre des systèmes étant réglées à 210 tours/mn.

Après deux jours d'agitation les fioles de préculture sont utilisées pour ensemencer les fioles de culture proprement dites à raison de 5 ml de milieu de préculture par Erlenmeyer de 500 ml garni de milieu de culture (100 ml) de composition :

| | |
|---|---|
| Glycérol | 10 g |
| Amidon soluble | 30 g |
| Soyoptim | 15 g |
| Tryptone | 2g |
| Extrait de levure | 5 g |
| CaCO₃ | 5 g |
| Solution d'oligoéléments | 10 ml |
| Eau QSP | 1 l |
| pH 7 | |

Composition de la solution d'oligoéléments utilisée :

| | |
|---|---|
| FeSO₄, 7 H₂O | 1,0 g |
| MnSO₄, 4 H₂O | 1,0 g |
| CaCl₂, 2 H₂O | 0,025 g |
| CaCl₂, 2 H₂O | 0,10g |
| H₃BO₃ | 0,56 g |
| (NH₄)₆ Mo₇O₂₄, 4 H₂O | 0,002 g |
| ZnSO₄, 7 H₂O | 0,20 g |
| Eau QSP | 1 l |

Ainsi dans ce cas précis, 5 fioles de précultures ont été utilisées pour inoculer 40 fioles de cultures à 100 ml de milieu de culture par Erlenmeyer de 500 ml, qui après 6 jours de culture agitée à 28° C en chambre chaude sur un agitateur rotatif réglé à 210 tours/mn, fournissent 4 litres de suspension de bactéries.

Les 4 litres de moût de fermentation sont centrifugés en plusieurs opérations, à une température de 4°C, et au régime de 13500 tours/mn (soit 27 500g avec le rotor utilisé), afin de séparer la biomasse, c'est à dire le culot rassemblant les cellules du surnageant de culture constitué majoritairement d'eau provenant du milieu nutritif utilisé et contenant en solution des restes de composants du milieu nutritif ainsi que des métabolites produits et excrétés par les cellules des bactéries pendant les diverses phases de leur croissance.

Les biomasses et sumageants sont alors congelés à -20° C.

### EXTRACTION DES LIGANDS NATURELS DU RECEPTEUR PBR

Les 4 litres de surnageant décongelé sont chargés dans un bécher de 10 litres. On additionne la solution de 400 g de résine polystyrène-divinylbenzène Amberlite XAD 16 (Rohm & Haas). La suspension est agitée avec un moteur équipé d'un arbre à pales, tournant à 20 tours/mn, pendant 15 heures. La solution est ensuite filtrée, le filtrat est éliminé et la résine essorée est reprise par 1 litre de méthanol. On laisse sous agitation douce pendant 1 heure. On filtre à nouveau la résine que l'on retraite à l'identique avec 1 litre de méthanol. Lors d'une troisième opération la résine est retraitée cette fois avec 1 litre d'acétone. La résine essorée est alors éliminée et les 3 litres de solvant organique rassemblés sont évaporés à sec dans un évaporateur rotatif sous vide.

Le résidu d'évaporation (17,7g) est trituré avec 50 ml de méthanol, la suspension obtenue est centrifugée à 3000 tours/mn pendant 15 minutes, et le surnageant décanté obtenu constitue l'extrait de surnageant de culture.

Cet extrait est testé en dilution en inhibition du binding sur le récepteur PBR et fournit une activité évaluée au 1/200 (50 % d'inhibition).

Les biomasses rassemblées (199g) dans un bêcher de 2 litres sont traitées sous agitation par un mélange de 750 ml de chlorure de méthylène et de 750 ml de méthanol. L'agitation est maintenue pendant 15 heures à température ambiante. La suspension est ensuite filtrée et la solution limpide obtenue est concentrée sous vide dans un évaporateur rotatif. Le résidu d'évaporation (5,4g) est alors trituré avec 50 ml de méthanol et constitue l'extrait de biomasse.

Cet extrait est testé en inhibition du binding du récepteur PBR et fournit une activité mesurée au 1/2200 (DI 50 = 2200⁻¹).

### EXEMPLE B SRL 5186

Avec les mêmes protocoles et milieux respectifs :
- milieu gélosé pour les repiquages sur boite de Pétri
- milieu liquide de préculture
- milieu liquide de production,
14 fioles d'Erlenmeyer de 500 ml garnies de 100 ml de milieu de production, et inoculées à 5 %, sont incubées à 28 °C en chambre chaude sur un agitateur rotatif tournant à 210 tours/mn, pendant 6 jours.

Après centrifugation et stockage un à deux jours au congélateur à - 20°C des biomasses et surnageants de production, on décongèle ces produits avant de procéder à leur extraction.

Les biomasses (54,9 g) sont traitées dans un bécher, sous agitation par un mélange de 250 ml de dichlorométhane et 250 ml de méthanol, pendant une dizaine d'heures. La suspension est ensuite filtrée et la solution limpide obtenue est concentrée à sec avec un évaporateur rotatif.

Le résidu sec (1,4 g) est trituré avec 17,5 ml de méthanol, et la suspension obtenue est centrifugée à 3000 tours/mn pendant 15 minutes. Le surnageant de centrifugation prélevé, constitue l'extrait de biomasse.

Cet extrait, évalué en dilution sur le test d'inhibition du binding au récepteur PBR fournit une inhibition de 50 % dans le test à la dilution 1/3750 (DI₅₀= 3750⁻¹).

Les 1400 ml de surnageant décongelé sont additionnés de 160 g de résine polystyrène-divinylbenzène XAD 16 (Rohm & Haas) et la suspension est agitée pendant 15 heures. La résine est filtrée, le filtrat est éliminé et la résine est retraitée par 200 ml de solution à 25 % de méthanol dans l'eau pendant 3 heures.

La résine est filtrée, et ce deuxième filtrat est éliminé. La résine subit alors trois traitements semblables, deux avec 200 ml de méthanol et le dernier avec 200 ml d'acétone. Ces trois derniers filtrats sont rassemblés dans un ballon puis concentrés sous vide à l'aide d'un évaporateur rotatif. Le résidu sec obtenu (2,2 g) est alors trituré avec 17,5 ml de méthanol et la solution obtenue constitue l'extrait de surnageant.

Cet extrait évalué en dilution sur le test d'inhibition du binding au récepteur PBR donne une inhibition de 50 % à la dilution 1/940 (DI₅₀ = 940⁻¹).

Dans les compositions selon l'invention, la substance se liant à PBR est utilisée de préférence en une quantité allant de 0,00001 à 20 % en poids par rapport au poids total de la composition, et en particulier en une quantité allant de 0,001 à 10 % en poids par rapport au poids total de la composition.

Les compositions selon l'invention peuvent se présenter sous toutes les formes galéniques normalement utilisées pour une application topique.

Les quantités des différents constituants des compositions selon l'invention sont celles classiquement utilisées dans les domaines considérés et sont appropriées à leur forme galénique.

Pour une application topique, les compositions de l'invention comprennent un milieu compatible avec la peau. Ces compositions peuvent se présenter notamment sous forme de solutions aqueuses, alcooliques ou hydroalcooliques, de gels, d'émulsions eau-dans-huile ou huile-dans-eau ayant l'aspect d'une crème ou d'un gel, de microémulsions, d'aérosols, ou encore sous forme de dispersions vésiculaires contenant des lipides ioniques et/ou non ioniques. Ces formes galéniques sont préparées selon les méthodes usuelles des domaines considérés.

Ces compositions à application topique peuvent constituer notamment une composition de protection, de traitement ou de soin cosmétique ou dermatologique pour le visage, pour le cou, pour les mains ou pour le corps, (par exemple crèmes de jour, crèmes de nuit, crèmes ou huiles solaires, laits corporels), une composition de maquillage (par exemple fond de teint) ou une composition de bronzage artificiel.

Quand la composition de l'invention est une émulsion, la proportion de corps gras qu'elle contient peut aller de 5 % à 80 % en poids, et de préférence de 5 % à 50 % en poids par rapport au poids total de la composition. Les corps gras et les émulsionnants utilisés dans la composition sous forme d'émulsion sont choisis parmi ceux classiquement utilisés dans le domaine cosmétique ou dermatologique.

Comme corps gras utilisables dans l'invention, on peut citer les huiles minérales (vaseline), les huiles végétales (fraction liquide de beurre de karité) et leurs dérivés hydrogénés, les huiles animales, les huiles de synthèse (perhydrosqualène), les huiles siliconées (diméthylpolysiloxane) et les huiles fluorées. Comme autres corps gras, on peut encore citer les alcools gras (alcool cétylique, alcool stéarylique), les acides gras (acide stéarique) et les cires.

Les émulsionnants peuvent être présents, dans la composition, en une proportion allant de 0,3 % à 30 % en poids et de préférence de 0,5 à 30 % en poids par rapport au poids total de la composition.

De façon connue, les compositions cosmétiques ou dermatologiques de l'invention peuvent contenir également des adjuvants habituels dans les domaines correspondants, tels que les gélifiants hydrophiles ou lipophiles, les conservateurs, les antioxydants, les solvants, les parfums, les charges, les filtres et les matières colorantes. Par ailleurs, ces compositions peuvent contenir des actifs hydrophiles ou lipophiles. Les quantités de ces différents adjuvants ou actifs sont celles classiquement utilisées dans le domaine cosmétique au dermatologique, et par exemple de 0,01 % à 20 % du poids total de la composition. Ces adjuvants ou ces actifs, selon leur nature, peuvent être introduits dans la phase grasse, dans la phase aqueuse et/ou dans des vésicules lipidiques.

Parmi les actifs que peuvent contenir les compositions de l'invention, on peut notamment citer les actifs ayant un effet sur le traitement des rides ou des ridules, et en particulier les actifs kératolytiques. Par kératolytique, on entend un actif ayant des propriétés desquamantes, exfoliantes ou gommantes, ou un actif capable de ramollir la couche cornée.

Parmi ces actifs ayant un effet sur le traitement des rides ou des ridules que peuvent contenir les compositions de l'invention, on peut en particulier citer les hydroxyacides et les rétinoïdes.

Les hydroxyacides peuvent être par exemple des α-hydroxyacides ou des β-hydroxyacides, qui peuvent être linéaires, ramifiés ou cycliques, saturés ou insaturés. Les atomes d'hydrogène de la chaîne carbonée peuvent, en outre, être substitués par des halogènes, des radicaux halogénés, alkylés, acylés, acyloxylés, alcoxy carbonylés ou alcoxylés ayant de 2 à 18 atomes de carbone.

Les hydroxyacides qui peuvent être utilisés sont notamment les acides glycolique, lactique, malique, tartrique, citrique, hydroxy-2 alcanoïque, mandélique, salicylique, ainsi que leurs dérivés alkylés comme l'acide n-octanoyl-5-salicylique, l'acide n-dodécanoyl-5-salicylique, l'acide n-décanoyl-5-salicylique, l'acide n-octyl-5-salicylique, l'acide n-heptyloxy-5 ou -4-salicylique, l'acide 2-hydroxy-3-méthylbenzoïque, ou encore leurs dérivés alcoxylés comme l'acide 2-hydroxy-3-méthoxybenzoïque.

Les rétinoïdes peuvent être notamment l'acide rétinoïque et ses dérivés, le rétinol (vitamine A) et ses esters tels que le palmitate de rétinol, l'acétate de rétinol et le propionate de rétinol, ainsi que leurs sels.

Ces actifs peuvent être utilisés en particulier à des concentrations allant de 0,0001 % à 5 % en poids par rapport au poids total de la composition.

## Revendications

1. Utilisation d'au moins une substance se liant au récepteur périphérique des benzodiazépines ou PBR, pour la fabrication d'une composition pharmacologique ou dermatologique topique dans le traitement du stress cutané.

2. Utilisation d'au moins une substance se liant au PBR pour la fabrication d'une composition pharmacologique et/ou dermatologique topique en vue de diminuer les rides, de réduire l'érythème solaire ou de protéger contre les radicaux libres.

3. Utilisation selon l'une des revendication 1 ou 2, **caractérisée en ce que** la substance se liant au PBR est un agoniste du PBR choisi parmi les molécules de synthèse, les substances naturelles d'extraction ou une substance obtenue par fermentation.

4. Utilisation selon l'une quelconque des revendications 1 à 3 **caractérisée en ce que** la substance se liant au PBR est le Ro 5-4864.

5. Utilisation selon l'une quelconque des revendications 1 à 3 **caractérisée en ce que** la substance se liant au PBR est une substance obtenue par fermentation.

6. Utilisation selon l'une quelconque des revendications 1 à 5, **caractérisée en ce que** la substance est présente dans la composition pharmacologique ou dermatologique en une quantité allant de 0,00001 à 20 % en poids par rapport au poids total de la composition.

7. Utilisation selon l'une quelconque des revendications 1 à 6 **caractérisée en ce que** la substance agoniste est présente dans la composition pharmacologique ou dermatologique en une quantité allant de 0,001 à 10 % en poids par rapport au poids total de la composition.

8. Utilisation selon l'une quelconque des revendications 1 à 7, **caractérisée en ce que** la composition contient en outre un hydroxyacide et/ou un rétinoïde.

9. Utilisation selon la revendication 8, **caractérisée en ce que** l'hydroxyacide est choisi parmi les α-hydroxyacides ou les β-hydroxyacides, qui peuvent être linéaires, ramifiés ou cycliques, saturés ou insaturés.

10. Utilisation selon la revendication 8, **caractérisée en ce que** le rétinoïde est choisi dans le groupe comprenant l'acide rétinoïque que et ses dérivés, le rétinol et ses esters.

11. Composition pharmacologique et/ou dermatologique topique **caractérisée en ce qu'**elle contient en tant que substance active une substance obtenue par fermentation de la souche Nocardia species I-2305 ou Streptomyces species I-2306.

## Patentansprüche

1. Verwendung von mindestens einer Substanz, die sich an den peripheren Rezeptor von Benzodiazepinen oder PBR bindet, zur Herstellung einer topischen pharmakologischen oder dermatologischen Zusammensetzung für die Behandlung von Hautstreß.

2. Verwendung von mindestens einer Substanz, die sich an PBR bindet, zur Herstellung einer topischen pharmakologischen und/oder dermatologischen Zusammensetzung im Hinblick auf die Verringerung von Falten, die Reduzierung von solarem Erythem oder den Schutz gegenüber freien Radikalen.

3. Verwendung nach Anspruch 1 oder 2, **dadurch gekennzeichnet, daß** die Substanz, die sich an PBR bindet, ein Agonist von PBR ist, ausgewählt unter den synthetischen Molekülen, den natürlichen Substanzen aus der Extraktion oder einer durch Fermentation erhaltenen Substanz.

4. Verwendung nach irgendeinem der Ansprüche 1 bis 3, **dadurch gekennzeichnet, daß** die Substanz, die sich an PBR bindet, Ro 5-4864 ist.

5. Verwendung nach irgendeinem der Ansprüche 1 bis 3, **dadurch gekennzeichnet, daß** die Substanz, die sich an PBR bindet, eine durch Fermentation erhaltene Substanz ist.

6. Verwendung nach irgendeinem der Ansprüche 1 bis 5, **dadurch gekennzeichnet, daß** die Substanz in der pharmakologischen oder dermatologischen Zusammensetzung in einer Menge anwesend ist, die von 0,00001 Gew.-% bis 20 Gew.-% reicht, bezogen auf das Gesamtgewicht der Zusammensetzung.

7. Verwendung nach irgendeinem der Ansprüche 1 bis 6, **dadurch gekennzeichnet, daß** die agonistische Substanz in der pharmakologischen oder dermatologischen Zusammensetzung in einer Menge anwesend ist, die von 0,001 Gew.-% bis 10 Gew.-% reicht, bezogen auf das Gesamtgewicht der Zusammensetzung.

8. Verwendung nach irgendeinem der Ansprüche 1 bis 7, **dadurch gekennzeichnet, daß** die Zusammensetzung außerdem eine Hydroxysäure und/oder ein Retinoid enthält.

9. Verwendung nach Anspruch 8, **dadurch gekennzeichnet, daß** die Hydroxysäure unter den α-Hydroxysäuren oder den β-Hydroxysäuren ausgewählt wird, die linear, verzweigt oder cyclisch, gesättigt oder ungesättigt sein können.

10. Verwendung nach Anspruch 8, **dadurch gekennzeichnet, daß** das Retinoid aus der Gruppe gewählt wird, die Retinoesäure und ihre Derivate, Retinol und seine Ester umfaßt.

11. Topische pharmakologische und/oder dermatologische Zusammensetzung, **dadurch gekennzeichnet, daß** sie als Wirkstoff eine Substanz umfaßt, die durch Fermentation des Stammes Nocardia Species 1-2305 oder Streptomyces Species 1-2306 erhalten wurde.

## Claims

1. Use of at least one peripheral benzodiazepine receptor- or PBR-binding substance, for producing a pharmacological or dermatological topical composition in the treatment of skin stress.

2. Use of at least one PBR-binding substance, for producing a pharmacological and/or dermatological topical composition for the purpose of decreasing wrinkles, of reducing solar erythema and of protecting against free radicals.

3. Use according to either of Claims 1 and 2, **characterized in that** the PBR-binding substance is a PBR agonist chosen from synthetic molecules, natural substances from extraction, or a substance obtained by fermentation.

4. Use according to any one of Claims 1 to 3, **characterized in that** the PBR-binding substance is Ro 5-4864.

5. Use according to any one of Claims 1 to 3, **characterized in that** the PBR-binding substance is a substance obtained by fermentation.

6. Use according to any one of Claims 1 to 5, **characterized in that** the substance is present in the pharmacological or dermatological composition in an amount ranging from 0.00001 to 20% by weight relative to the total weight of the composition.

7. Use according to any one of Claims 1 to 6, **characterized in that** the agonist substance is present in the pharmacological or dermatological composition in an amount ranging from 0.001 to 10% by weight relative to the total weight of the composition.

8. Use according to any one of Claims 1 to 7, **characterized in that** the composition also contains a hydroxy acid and/or a retinoid.

9. Use according to Claim 8, **characterized in that** the hydroxy acid is chosen from α-hydroxy acids or β-hydroxy acids, which may be linear, branched or cyclic, and saturated or unsaturated.

10. Use according to Claim 8, **characterized in that** the retinoid is chosen from the group comprising retinoic acid and its derivatives, and retinol and its esters.

11. Pharmacological and/or dermatological topical composition, **characterized in that** it contains, as active substance, a substance obtained by fermentation of the strain Nocardia, species 1-2305, or Streptomyces, species 1-2306.
